# EUROPÄISCHE PATENTANMELDUNG

(11) **EP 0 938 868 A1**
(43) Veröffentlichungstag der Anmeldung: **01.09.1999**
(21) Anmeldenummer: 99810140.6
(22) Anmeldetag: 17.02.1999
(51) Int. Cl.: A61B 8/06

(54) **Nicht-invasive Blutflussmessanordnung**

(30) Priorität: 26.02.1998 EP 98810156
(71) Anmelder: Schiller, Alfred, 8914 Aeugst a.A. (CH)
(72) Erfinder: Schiller, Alfred, 8914 Aeugst a.A. (CH)
(74) Vertreter: Müller, Christoph Emanuel

(57) **Zusammenfassung**

Eine Anordnung zum Messen des Blutflusses aus einer Vielzahl von nebeneinander angeordneten einzelnen Flussmesseinheiten (11, 11'). Die Vielzahl der nebeneinander angeordneten einzelnen Flussmesseinheiten erlaubt ein rasches Aufsetzen der Messanordnung. Unabhängig vom genauen Ort am menschlichen Körper, an dem die Messanordnung plaziert wird, liegt immer eine Schlagader, insbesondere die Halsschlagader benachbart zu wenigstens einer der Flussmesseinheiten.

## Beschreibung

Die Erfindung betrifft eine Messanordnung mit den Merkmalen des Patentanspruch 1.

Es ist bekannt, bei Herzstillstand oder bei Herzstörungen Elektroschocks oder Herzmassagen einzusetzen, um die Herztätigkeit wieder anzuregen. Defibrillatoren (zum Erzeugen von Elektroschocks) oder Herzmassagegeräte werden häufig bei Erste-Hilfeleistungen eingesetzt.

Es ist wichtig zu wissen, zu welchem Zeitpunkt und ob eine Defibrillation bzw. eine Herzmassage durchgeführt werden muss. Bei einsetzendem Herzschlag sollten weitere Herzschläge durch gezielte weitere Stromstösse oder Massagen unterstützt werden. Eine Defibrillation sollte nur durchgeführt werden, wenn beim Patienten kein Puls mehr festgestellt wird. Normalerweise wird dabei der (Carotis-) Puls des Patienten manuell getastet. Dabei kann es vor allem bei schwachem Puls zu einer Überlagerung des Pulses des Patienten mit dem Puls des Helfers kommen.

Die Durchblutung des Kopfes muss innerhalb von drei Minuten nach einem Herzstillstand wieder hergestellt werden, um irreparable Hirnschäden zu vermeiden. Es ist deshalb nötig, möglichst rasch den Puls bestimmen zu können.

Bei allen Anwendungen von Defibrillationen wird die mechanische Tätigkeit des Herzens erfasst. Weil häufig in den Extremitäten des Patienten Blutleere herrscht, wird der Carotispuls an der Halsschlagader gemessen.

Vor allem bei Erste-Hilfemassnahmen ist es wichtig, dass mit dem Bestimmen des Pulses keine Zeit verloren wird. Nicht nur bei Reanimation durch Herzmassage oder Defibrillation, sondern auch in anderen Situationen kann die rasche und zuverlässige Bestimmung des Pulses von entscheidender Bedeutung sein.

Es ist eine Aufgabe der vorliegenden Erfindung, die Nachteile des Bekannten zu vermeiden und eine Anordnung zum Bestimmen des Pulses zu schaffen, welche ein mühsames Suchen der Schlagader vermeidet und welche eine rasche Bestimmung des Pulses ermöglicht.

Erfindungsgemäss wird diese Aufgabe mit einer Anordnung mit den Merkmalen des unabhängigen Patentanspruchs 1 gelöst.

Die Erfindung betrifft in erster Linie eine Flussmessanordnung, welche sich insbesondere zum Bestimmen des Pulses eignet. Selbstverständlich ist die nachfolgend beschriebene erfinderische Idee in anderen Messverfahren, bei welchen es auf eine rasche Messung ankommt, ebenfalls vorteilhaft einsetzbar.

Erfindungsgemäss beruht die Anordnung zum Messen des Pulses auf einer Flussmessung. Durch Messung des Blutflusses, insbesondere in den Kopf, kann der Puls eines Patienten bestimmt werden.

Unter Blutfluss wird in diesem Zusammenhang das Blutvolumen verstanden, das pro bestimmte Zeiteinheit vom Herz in die Kopfpartie gepumpt wird. Der Blutfluss kann am einfachsten an der Halsschlagader gemessen werden.

Zum Messen des Blutflusses können grundsätzlich beliebige Flussmessanordnungen verwendet werden. Besonders vorteilhaft ist eine auf dem Dopplereffekt basierende Messzelle. Dopplermessungen zum Bestimmen der Geschwindigkeit des Blutes in Blutgefässen sind zu diagnostischen Zwecken bereits bekannt.

Weil bei einem Herzstillstand die Reanimation möglichst schnell begonnen werden muss, sollte keine Zeit verloren werden, um die Halsschlagader zum Aufsetzen der Anordnung zum Messen des Blutflusses zu lokalisieren. Gemäss der vorliegenden Erfindung besteht die Anordnung zum nicht-invasiven Messen des Blutflusses aus einer Vielzahl von nebeneinander angeordneten Flussmesseinheiten. Die Signalausgänge der einzelnen Flussmesseinheiten sind mit einer Signalverarbeitungsanordnung gekoppelt. Die relative Lage, die Anzahl und/oder die Grösse der Flussmesseinheiten ist dabei derart gewählt, dass selbst bei etwa im rechten Winkel auf die Schlagader aufgesetzter Messanordnung in jeder Aufsetzlage wenigstens eine Flussmesseinheit ausreichend nah der Schlagader liegt, dass ein messbares Messsignal erzeugt wird. Etwa rechtwinklig bedeutet in diesem Fall, dass die nebeneinanderliegenden Messeinheiten quer auf die Schlagader aufgesetzt werden. Dank dieser Ausführungsform kann die erfindungsgemässe Messanordnung auf den Hals des Patienten gelegt werden, ohne dass ein exaktes Aufsetzen nötig ist. Unabhängig davon, wie die Anordnung auf den Hals des Patienten aufgelegt wird, ist immer eine Flussmesseinheit richtig auf der Halsschlagader positioniert.

Es versteht sich von selbst, dass eine solche Messanordnung bei Reanimation durch eine Defibrillation oder Herzmassage besonders vorteilhaft einsetzbar ist. Es ist aber durchaus auch denkbar, die Messanordnung in anderen Anwendungsgebieten einzusetzen, wo ein rasches Bestimmen des Blutflusses nötig ist.

Vorteilhaft weist die Messanordnung etwa 3 bis 10 nebeneinander angeordnete Messeinheiten auf. Jede Messeinheit weist eine Oberfläche mit ca. 4 mm Durchmesser bis 8 mm Durchmesser auf. Als Messeinheiten werden bevorzugt auf der Messung des Dopplereffekts basierende piezoelektrische Messzellen eingesetzt. Es ist aber auch denkbar, andere bekannte Flussmesseinheiten in der erfindungsgemässen Art und Weise anzuordnen.

Vorteilhaft wird eine Reihe von nebeneinander liegenden Transducerelementen verwendet. Es können sowohl im gepulsten oder im continuous wave Verfahren arbeitende Transducer eingesetzt werden.

Alternativ ist es denkbar, zwei Reihen versetzt zueinander angeordnete Transducer vorzusehen. Damit kann die Wahrscheinlichkeit, dass in jedem Fall genau ein Transducer auf die Halsschlagader zu liegen kommt, auf nahezu 100 % vergrössert werden.

Die Erfindung wird im folgenden in Ausführungsbeispielen und anhand der Zeichnungen näher erläutert. Es zeigen:
- Figur 1: eine schematische Darstellung einer erfindungsgemässen Messanordnung,
- Figur 2: ein alternatives Ausführungsbeispiel einer Messanordnung,
- Figur 3: ein weiteres Ausführungsbeispiel einer Messanordnung,
- Figur 4: die Messanordnung gemäss Figur 2 im Querschnitt entlang einer Linie A-A, und
- Figur 5: eine schematische Darstellung der Messanordnung in Anwendung an einem Patienten.

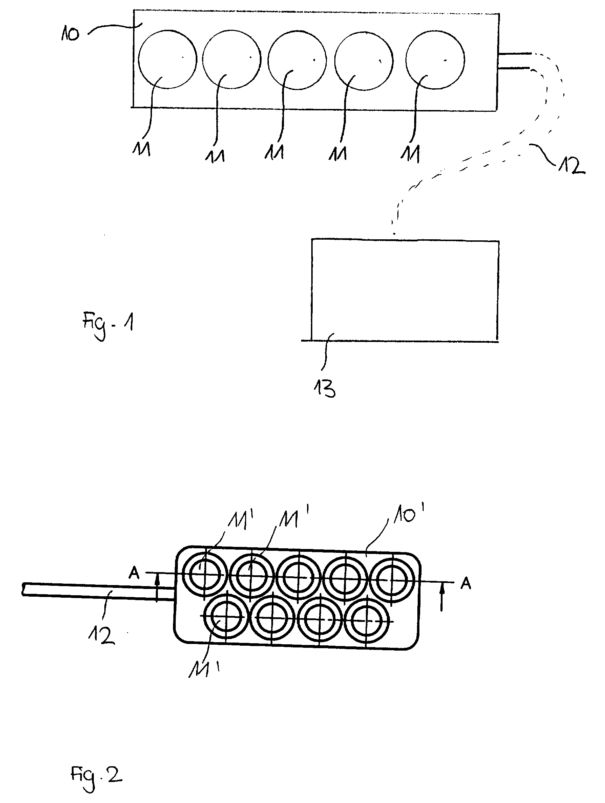

Figur 1 zeigt schematisch eine erfindungsgemässe Messanordnung 10 zum nicht-invasiven Messen des Blutflusses. Die Messanordnung 10 besteht aus fünf nebeneinander angeordneten Flussmesseinheiten 11. Die Ausgänge 12 der Flussmesseinheiten 11 sind mit einer Signalverarbeitungsanordnung 13 gekoppelt.

Die Flussmesseinheiten 11 haben einen Durchmesser von 4 bis 5 mm. Es werden auf dem Prinzip der Dopplereffektmessung basierende Messeinheiten im gepulsten oder im continuous wave Verfahren verwendet. Je nach Einsatzzweck variert der Frequenzbereich der Transducer. Zum Messen des Carotis-Pulses wird eine Frequenz von 8 MHz verwendet. Zum Bestimmen des Pulses an tiefer liegenden Arterien können Frequenzen von 4 oder 2 MHz verwendet werden.

Typischerweise weist jede Flussmesseinheit 11 einen Sender und einen Empfänger auf (je piezoelektrische Elemente). Der Sender erzeugt kontinuierlich Schwingungen. Der Empfänger erfasst die an den Blutplättchen reflektierten Schallwellen und berechnet (in bekannter Weise) aufgrund der Frequenzverschiebung (Dopplereffekt) die Geschwindigkeit des Blutes. Dies heisst, dass in erster Linie die Geschwindigkeit des Blutes gemessen wird. Der Wert des Blutflusses ist proportional zur Geschwindigkeit. Das Signal des Empfängers wird über einen Vorverstärker verstärkt und in die Signalverarbeitungsanordnung 13 geleitet. Typischerweise wird der Ausgang jedes Empfängers in einen Vorverstärker gegeben. Die Ausgänge des Vorverstärkers sind mit einem handelsüblichen Chip mit mehreren Eingängen gekoppelt (z.B. H8 von Hitachi). Der Chip ermittelt das stärkste Signal und erzeugt ein der Stärke des stärksten Signals entsprechendes Ausgangssignal.

Wenn die erfindungsgemässe Messanordnung 10 beispielsweise auf den Hals eines Patienten (siehe Fig. 5) gelegt wird, liegt immer eine der Flussmesseinheiten 11 auf oder benachbart zur Halsschlagader. Diese Flussmesseinheit 11a erzeugt ein Signal, welches den Wert des Blutflusses wiedergibt. Die übrigen Flussmesseinheiten, welche nicht direkt benachbart zu der Halsschlagader liegen, erzeugen ein schwächeres oder kein Signal.

Selbstverständlich ist es auch denkbar, mit einer Signalverarbeitungsanordnung 13 die Messwerte aller Flussmesseinheiten 11 zu berücksichtigen und ein integriertes Signal zu berechnen. Dies kann vor allem bei Anordnungen vorteilhaft sein, bei welchen die Dimensionen der Flussmesseinheiten 11 so gewählt sind, dass mehr als eine Flussmesseinheit 11 gleichzeitig benachbart zu der Halsschlagader liegt.

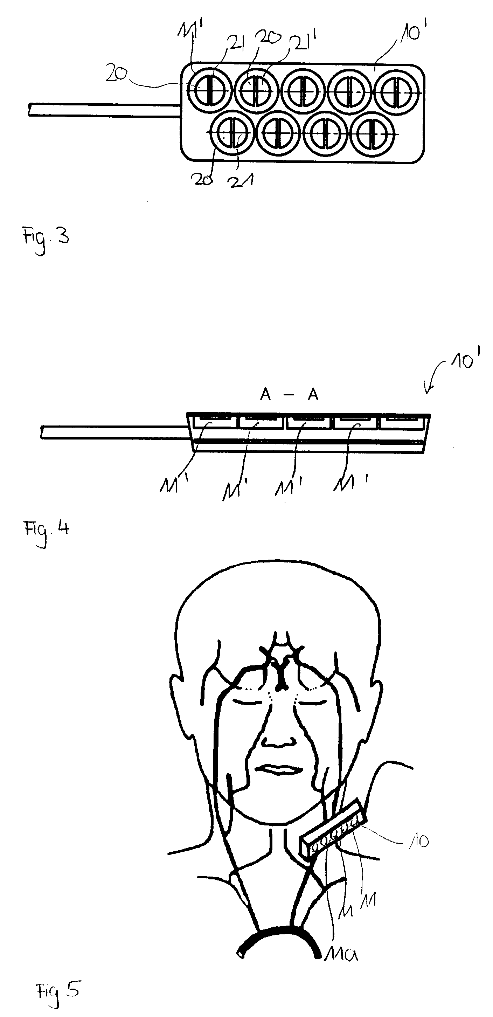

In Figur 2 und 3 ist ein alternatives Ausführungsbeispiel einer Messanordnung 10' gezeigt. Die einzelnen Flussmesseinheiten 11' sind in zwei Reihen versetzt zueinander angeordnet. Damit kann die Auflösung erhöht beziehungsweise die Wahrscheinlichkeit vergrössert werden, dass eine Flussmesseinheit jeweils genau auf der Schlagader liegt. Gemäss dem Ausführungsbeispiel in Figur 2 bestehen die Flussmesseinheiten 11 aus gepulsten Transducer-Elementen, welche gleichzeitig senden und empfangen.

In Figur 3 bestehen die Flussmesseinheiten 11 je aus einem Sender 20 und einem Empfänger 21. Sender 20 und Empfänger 21 sind im Continuous Wave-Verfahren betriebene Transducer-Elemente.

Die einzelnen Flussmesseinheiten 11, 11' können in Silikongummi eingegossen oder auch starr miteinander verbunden sein.

Figur 4 zeigt die Messanordnung 10' gemäss Figur 2 im Querschnitt. Fünf Flussmesseinheiten 11' sind nebeneinander angeordnet und liegen im wesentlichen in einer Ebene.

Während vorangehend der Einsatz der erfindungsgemässen Messanordnung vor allem im Zusammenhang mit Erste-Hilfemassnahmen erläutert wurde, ist eine Messanordnung mit einer Vielzahl von nebeneinanderliegend angeordneten Messeinheiten selbstverständlich auch in anderen Anwendungszwecken, wo es auf das rasche Bestimmen eines Messignals ankommt, einsetzbar. Der Grundgedanke der Erfindung besteht darin, dass eine Vielzahl von einzelnen Messzellen verwendet wird, von welchen unabhängig von der Art und Weise des Aufsetzens der Messanordnung immer wenigstens eine ein Signal erzeugt.

## Patentansprüche

1. Messanordnung (10, 10') insbesondere zum nicht-invasiven Messen des Blutflusses (B) mit einer Vielzahl von nebeneinander angeordneten Messeinheiten, insbesondere Flussmesseinheiten (11, 11'), deren Ausgänge (12) mit einer Signalverarbeitungsanordnung (8, 13) gekoppelt sind, dadurch gekennzeichnet, dass die relative Lage, die Anzahl und/oder die Grösse der Flussmesseinheiten derart gewählt ist, dass bei auf eine Schlagader, insbesondere auf die Halsschlagader aufgesetzter Messanordnung in jeder Aufsetzlage wenigstens eine Flussmesseinheit derart benachbart zu der Schlagader liegt, dass ein messbares Messsignal erzeugbar ist.

2. Anordnung nach Anspruch 1, dadurch gekennzeichnet, dass die Anordnung (10) 4 bis 10 Flussmesseinheiten (11) aufweist.

3. Messanordnung nach einem der Ansprüche 1 oder 2, dadurch gekennzeichnet, dass jede Flussmesseinheit einen Durchmesser von 0,5 bis 1 cm aufweist.

4. Messanordnung nach einem der Ansprüche 1 bis 3, dadurch gekennzeichnet, dass die Flussmesseinheiten (11, 11') im continuous wave Verfahren arbeitende Dopplereffekt-Messeinheiten sind.

5. Messanordnung nach einem der Ansprüche 1 bis 3, dadurch gekennzeichnet, dass die Flussmesseinheiten (11, 11') im gepulsten Verfahren arbeitende Dopplereffekt-Messeinheiten sind.

6. Anordnung nach einem der Ansprüche 1 bis 5, dadurch gekennzeichnet, dass die Flussmesseinheiten in einer Reihe nebeneinanderliegend angeordnet sind.

7. Anordnung nach einem der Ansprüche 1 bis 5, dadurch gekennzeichnet, dass die Flussmesseinheiten in zwei Reihen versetzt zueinander angeordnet sind.

8. Verfahren zum nicht-invasiven Messen des Blutflusses in einer Schlagader, dadurch gekennzeichnet, dass eine Mehrzahl von Flussmesseinheiten im Bereich der Schlagader aufgelegt werden, wobei in jeder Flussmesseinheit ein Ausgangssignal erzeugt wird, wobei die Ausgangssignale vorzugsweise verstärkt werden und wobei aus den Ausgangssignalen der Wert des Blutflusses, insbesondere aus dem stärksten Ausgangssignal, ermittelt wird.
